(19) Europäisches Patentamt
European Patent Office
Office européen des brevets

(11) Veröffentlichungsnummer : **0 032 200**
**B1**

(12) # EUROPÄISCHE PATENTSCHRIFT

(45) Veröffentlichungstag der Patentschrift : **13.07.83**

(51) Int. Cl.³ : **A 01 N 43/64**, C 07 D249/08

(21) Anmeldenummer : **80107698.5**

(22) Anmeldetag : **06.12.80**

(54) **Substituierte Triazolylmethyl-tert.-butyl-carbinole, Verfahren zu ihrer Herstellung und ihre Verwendung als Pflanzenschutzmittel sowie als Zwischenprodukte.**

(30) Priorität : **19.12.79 DE 2951163**

(43) Veröffentlichungstag der Anmeldung : **22.07.81 Patentblatt 81/29**

(45) Bekanntmachung des Hinweises auf die Patenterteilung : **13.07.83 Patentblatt 83/28**

(84) Benannte Vertragsstaaten : **AT BE CH DE FR GB IT LI NL SE**

(56) Entgegenhaltungen :
**EP A 0 004 303**
**EP A 0 015 639**
**DE A 2 734 426**
**DE A 2 737 489**

**Die Akte enthält technische Angaben, die nach dem Eingang der Anmeldung eingereicht wurden und die nicht in dieser Patentschrift enthalten sind.**

(73) Patentinhaber : **BAYER AG**
**Zentralbereich Patente, Marken und Lizenzen**
**D-5090 Leverkusen 1, Bayerwerk (DE)**

(72) Erfinder : **Krämer, Wolfgang, Dr.**
**Am Eckbusch 39/45**
**D-5600 Wuppertal 1 (DE)**
Erfinder : **Büchel, Karl Heinz, Prof. Dr.**
**Dabringhausener Strasse 42**
**D-5093 Burscheid (DE)**
Erfinder : **Brandes, Wilhelm, Dr.**
**Eichendorffstrasse 3**
**D-5653 Leichlingen 1 (DE)**
Erfinder : **Lürssen, Klaus, Dr.**
**August-Kierspel-Strasse 89**
**D-5060 Bergisch Gladbach 2 (DE)**
Erfinder : **Frohberger, Paul-Ernst, Dr.**
**Willi-Baumeister-Strasse 5**
**D-5090 Leverkusen (DE)**

**EP 0 032 200 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Substituierte Triazolylmethyl-tert.-butyl-carbinole, Verfahren zu ihrer Herstellung und ihre Verwendung als Pflanzenschutzmittel sowie als Zwischenprodukte

Die vorliegende Erfindung betrifft neue substituierte Triazolylmethyl-tert.-butyl-carbinole, ein Verfahren zu ihrer Herstellung sowie ihre Verwendung als Pflanzenschutzmittel und als Zwischenprodukte für die Synthese von weiteren Pflanzenschutzmitteln.

Aus der DE-A 27 34 426 und der DE-A 27 37 439 sind bereits zahlreiche Triazolyl-tert.-butyl-ketone und Triazolyl-tert.-butyl-carbinole mit pflanzenwuchsregulierenden und fungiziden Eigenschaften bekannt. Entsprechende Stoffe, die eine durch Halogen substituierte tert.-Butyl-Gruppe enthalten, wurden jedoch bisher noch nicht offenbart. Von den in der DE-A 27 34 426 und der DE-A 27 37 489 beschriebenen Stoffen lassen sich beispielsweise 1-(4-Chlorphenyl)-4,4-dimethyl-2-(1,2,4-triazol-1-yl)-pentan-3-on, 1-(4-Chlorphenyl)-4,4-dimethyl-2-(1,2,4-triazol-1-yl)-pentan-3-ol und 1-Phenyl-4,4-dimethyl-2-(1,2,4-triazol-1-yl)-pentan-3-ol als Fungizide und Pflanzenwuchsregulatoren verwenden. Die Wirkung dieser Triazol-Derivate läßt allerdings insbesondere bei niedrigen Aufwandmengen zu wünschen übrig.

Weiterhin ist bereits bekannt geworden, daß bestimmte Triazolyl-keton-Derivate gute fungizide Wirksamkeit besitzen. So kann zum Beispiel das 1-(2,4-Dichlorphenoxy)-3,3-dimethyl-1-(1,2,4-triazol-1-yl)-butan-2-on zur Bekämpfung von Pilzen eingesetzt werden (vgl. DE-B 22 01 063). Auch die Wirkung dieses Stoffes ist jedoch nicht immer ganz befriedigend.

Es wurden nun neue substituierte Triazolylmethyl-tert.-butyl-carbinole der Formel

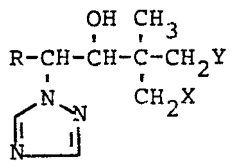

(I)

in welcher

R für Alkyl mit 1 bis 12 Kohlenstoffatomen, Alkenyl mit 2 bis 12 Kohlenstoffatomen, Alkinyl mit 2 bis 12 Kohlenstoffatomen, gegebenenfalls durch Alkyl mit 1 bis 4 Kohlenstoffatomen substituiertes Cycloalkyl mit 3 bis 7 Kohlenstoffatomen, gegebenenfalls durch Alkyl mit 1 bis 4 Kohlenstoffatomen substituiertes Cycloalkylalkyl mit 3 bis 7 Kohlenstoffatomen im Cycloalkylteil und 1 bis 4 Kohlenstoffatomen im Alkylteil sowie für Aralkyl mit 6 bis 10 Kohlenstoffatomen im Arylteil und 1 bis 4 Kohlenstoffatomen im Alkylteil steht, wobei der Arylteil substituiert sein kann durch Alkyl mit 1 bis 4 Kohlenstoffatomen, Alkoxy mit 1 bis 4 Kohlenstoffatomen, Alkylthio mit 1 bis 4 Kohlenstoffatomen, Halogen, Halogenalkyl mit 1 bis 4 Kohlenstoffatomen und 1 bis 5 gleichen oder verschiedenen Halogenatomen, Halogenalkoxy mit 1 bis 4 Kohlenstoffatomen und 1 bis 5 gleichen oder verschiedenen Halogenatomen, Halogenalkylthio mit 1 bis 4 Kohlenstoffatomen und 1 bis 5 gleichen oder verschiedenen Halogenatomen, Nitro, Cyano, Alkoxycarbonyl mit 1 bis 4 Kohlenstoffatomen im Alkoxyteil, Amino, Alkylamino mit 1 bis 4 Kohlenstoffatomen, Dialkylamino mit 1 bis 4 Kohlenstoffatomen in jedem Alkylteil, Phenylamino, Phenoxy und/oder Phenyl, welches seinerseits substituiert sein kann durch Halogen und/oder Alkyl mit 1 bis 2 Kohlenstoffatomen,

X für Wasserstoff steht und

Y für Fluor oder Chlor steht oder

X und Y gleich sind und für Fluor oder Chlor stehen,

sowie deren pflanzenverträgliche Säureadditions-Salze und Metallsalz-Komplexe gefunden.

Die Verbindungen der Formel (I) besitzen zwei asymmetrische Kohlenstoffatome; sie können deshalb in den beiden geometrischen Isomeren (threo- und erythro-Form) vorliegen, die in unterschiedlichen Mengenverhältnissen anfallen können. In beiden Fällen liegen sie als optische Isomere vor. Sämtliche Isomeren werden erfindungsgemäß beansprucht.

Weiterhin wurde gefunden, daß man die substituierten Triazolylmethyl-tert.-butyl-carbinole der Formel (I) sowie deren pflanzenverträgliche Säureadditions-Salze und Metallsalz-Komplexe erhält, wenn man substituierte Triazolylmethyl-tert.-butyl-ketone der Formel

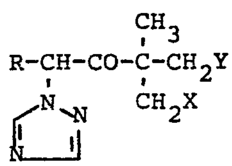

(II)

in welcher R, X und Y die oben angegebene Bedeutung haben, nach bekannten Methoden in üblicher Weise reduziert und gegebenenfalls anschließend eine Säure oder ein Metallsalz addiert.

Die neuen substituierten Triazolylmethyl-tert.-butyl-carbinole der Formel (I) sowie deren Säureadditions-Salze und Metallsalz-Komplexe weisen starke fungizide und pflanzenwachstumsregulierende Eigenschaften auf und sind daher als Pflanzenschutzmittel verwendbar.

Ueberraschenderweise zeigen die erfindungegemäßen substituierten Triazolylmethyl-tert.-butyl-carbinole eine bessere fungizide Wirkung als die aus dem Stand der Technik bekannten Triazolyl-Derivate 1-(2,4-Dichlorphenoxy)-3,3-dimethyl-1-(1,2,4-triazol-1-yl)-butan-2-on sowie 1-(4-Chlorphenyl)-4,4-dimethyl-2-(1,2,4-triazol-1-yl)-pentan-3-on und -ol, die chemisch und wirkungsmäßig naheliegende Verbindungen sind. Sie übertreffen ferner auch das 1-(4-Chlorphenyl)-4,4-dimethyl-2-(1,2,4-triazol-1-yl)-pentan-3-ol und das 1-Phenyl-4,4-dimethyl-2-(1,2,4-triazol-1-yl)-pentan-3-ol bezüglich ihrer pflanzenwuchsregulierenden Eigenschaften, was auf Grund des bekannten Standes der Technik nicht vorauszusehen war.

Außerdem sind die neuen substituierten Triazolylmethyl-tert.-butyl-carbinole interessante Zwischenprodukte zur Herstellung von weiteren Pflanzenschutz-Wirkstoffen.

Besonders bevorzugt sind diejenigen Verbindungen der Formel (I), in denen R für Methyl, Ethyl, n-Propyl, Isopropyl, n-Butyl, Isobutyl, sec.-Butyl, tert.-Butyl, Vinyl, Allyl, Butenyl, Propargyl, Butinyl, Cyclohexyl, Cyclohexylmethyl, sowie für Benzyl oder Naphthylmethyl steht, die gegebenenfalls einfach oder mehrfach, gleich oder verschieden substituiert sein können durch Methyl, Ethyl, Isopropyl, Methoxy, Methylthio, Fluor, Chlor, Trifluormethyl, Trifluormethoxy, Trifluormethylthio, Nitro, Cyano, Phenyl oder Phenoxy ; und X und Y für die oben genannten Reste stehen.

Im einzelnen seien außer den bei den Herstellungsbeispielen genannten Verbindungen folgende Verbindungen der allgemeinen Formel (I) genannt :

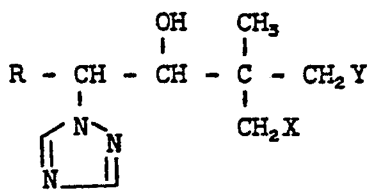

(I)

| X | Y | R |
|---|---|---|
| H | F | $C_2H_5$ |
| H | F | $i\text{-}C_3H_7$ |
| H | F | $C_4H_9$ |
| H | F | $-CH_2-CH=CH_2$ |
| H | F | $-CH_2-C\equiv CH$ |
| H | F | 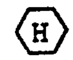 |
| H | F | 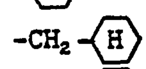 |
| H | F | 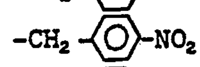 |
| H | F | 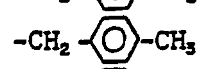 |
| H | F | 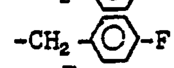 |
| H | F | 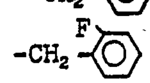 |
| H | F | 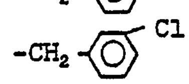 |
| H | F | 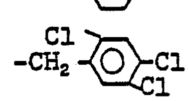 |
| H | F | 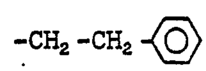 |
| H | F | 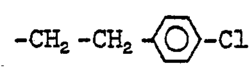 |
| H | F | 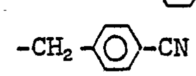 |
| H | F | 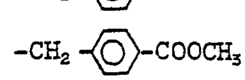 |
| H | Cl | $CH_3$ |

(Fortsetzung)

| X | Y | R |
|---|---|---|
| H | Cl | $C_2H_5$ |
| H | Cl | $i-C_3H_7$ |
| H | Cl | $C_4H_9$ |
| H | Cl | $-CH_2-CH=CH_2$ |
| H | Cl | $-CH_2-C\equiv CH$ |
| H | Cl | 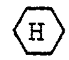 |
| H | Cl | 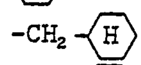 |
| H | Cl | 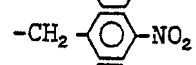 |
| H | Cl | 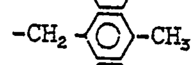 |
| H | Cl | 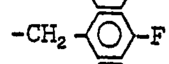 |
| H | Cl | 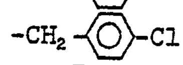 |
| H | Cl | 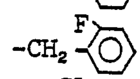 |
| H | Cl | 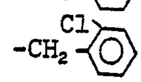 |
| H | Cl | 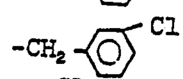 |
| H | Cl | 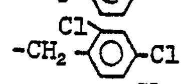 |
| H | Cl | 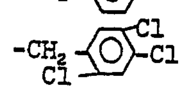 |
| H | Cl | 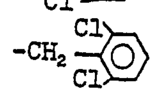 |
| H | Cl | 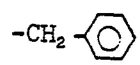 |
| Cl | Cl | $CH_3$ |
| Cl | Cl | $C_2H_5$ |
| Cl | Cl | $i-C_3H_7$ |
| Cl | Cl | $C_4H_9$ |
| Cl | Cl | $-CH_2-CH=CH_2$ |
| Cl | Cl | $-CH_2-C\equiv CH$ |
| Cl | Cl | 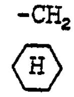 |
| Cl | Cl | 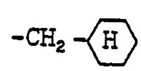 |
| Cl | Cl | 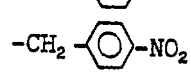 |
| Cl | Cl | 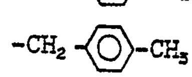 |
| Cl | Cl | 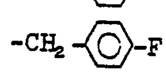 |
| Cl | Cl | 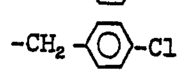 |

(Fortsetzung)

| X | Y | R |
|---|---|---|
| Cl | Cl | 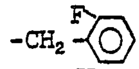 |
| Cl | Cl | 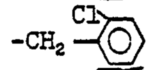 |
| Cl | Cl | 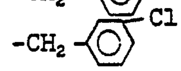 |
| Cl | Cl | 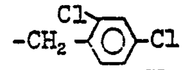 |
| Cl | Cl | 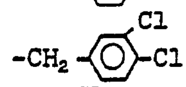 |
| Cl | Cl | 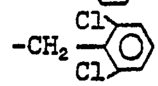 |
| Cl | Cl | 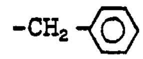 |
| F | F | $CH_3$ |
| F | F | $C_2H_5$ |
| F | F | $i-C_3H_7$ |
| F | F | $C_4H_9$ |
| F | F | $-CH_2-CH=CH_2$ |
| F | F | $-CH_2-C\equiv CH$ |
| F | F | 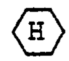 |
| F | F | 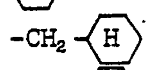 |
| F | F | 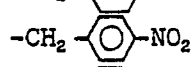 |
| F | F | 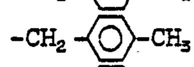 |
| F | F | 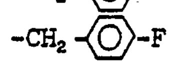 |
| F | F | 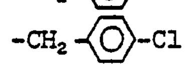 |
| F | F | 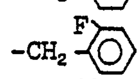 |
| F | F | 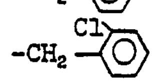 |
| F | F | 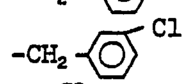 |
| F | F | 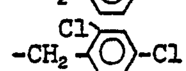 |
| F | F | 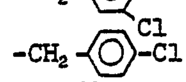 |
| F | F | 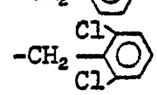 |
| F | F | 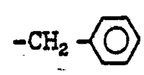 |

Verwendet man beispielsweise 1-Fluor-2,2-dimethyl-4-(1,2,4-triazol-1-yl)-pentan-3-on und Natriumborhydrid als Ausgangsstoffe, so kann der Reaktionsablauf durch das folgende Formelschema wiedergegeben werden :

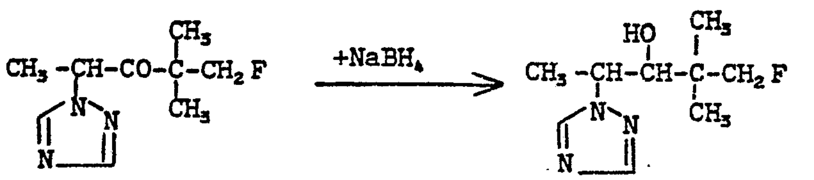

Die für das erfindungsgemäße Verfahren als Ausgangsstoffe zu verwendenden substituierten Triazolylmethyl-tert.-butyl-ketone sind durch die Formel (II) allgemein definiert. In dieser Formel stehen R, X und Y für die Reste, die bei den Verbindungen der Formel (I) bereits für diese Substituenten genannt wurden.

Die substituierten Triazolylmethyl-tert.-butyl-ketone der Formel (II) sind noch nicht bekannt ; sie sind jedoch Gegenstand einer eigenen parallelen Anmeldung und werden erhalten, indem man Triazolylmethyl-tert.-butyl-ketone der Formel

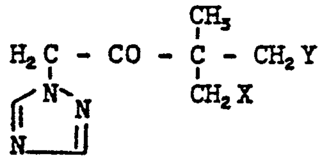

(III)

in welcher X und Y die oben angegebene Bedeutung haben, mit einem Alkylierungsmittel der Formel

$$R - Z \qquad \text{(IV)}$$

in welcher

R die oben angegebene Bedeutung hat und

Z für eine elektronenanziehende Abgangsgruppe, wie Halogen, p-Methylphenyl-sulfonyloxy oder Sulfat steht,

in üblicher Weise in Gegenwart eines inerten organischen Lösungsmittels, wie beispielsweise Dimethylsulfoxid, bei Temperaturen zwischen 0 und 100 °C umsetzt (vergleiche auch die Herstellungsbeispiele).

Die Triazolylmethyl-tert.-butyl-ketone der Formel (III) sind bekannt oder lassen sich nach im Prinzip bekannten Verfahren herstellen (vgl. DE-OS 28 20 361). Man erhält die Verbindungen der Formel (III), indem man Halogenketone der Formel

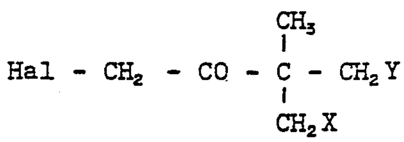

(V)

in welcher

X und Y die oben angegebene Bedeutung haben und

Hal für Chlor oder Brom steht,

mit 1,2,4-Triazol in Gegenwart eines Verdünnungsmittels, wie beispielsweise Aceton, und in Gegenwart eines Säurebindemittels, wie beispielsweise Kaliumcarbonat, bei Temperaturen zwischen 20 und 150 °C umsetzt.

Die Halogenketone der Formel (V) werden erhalten, indem man Verbindungen der Formel

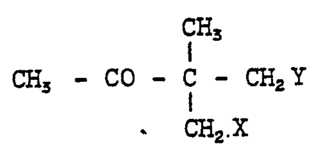

(VI)

in welcher X und Y die oben angegebene Bedeutung haben,

in einem inerten organischen Lösungsmittel bie Raumtemperatur mit Chlor oder Brom versetzt ; oder z. B. mit üblichen Chlorierungsmitteln, wie Sulfurylchlorid, bei 20 bis 60 °C umsetzt (vergleiche auch die Herstellungsbeispiele).

Die erfindungsgemäße Reduktion erfolgt in üblicher Weise, wie z. B. durch Umsetzung mit komplexen Hydriden, gegebenenfalls in Gegenwart eines Verdünnungsmittels ; oder durch Umsetzung mit Aluminiumisopropylat in Gegenwart eines Verdünnungsmittels ; oder durch Umsetzung mit Wasserstoff in Gegenwart eines Katalysators und gegebenenfalls in Gegenwart eines Verdünnungsmittels.

Arbeitet man mit komplexen Hydriden, so kommen als Verdünnungsmittel für die erfindungsgemäße Umsetzung polare organische Lösungsmittel infrage. Hierzu gehören vorzugsweise Alkohole, wie Methanol, Ethanol, Butanol, Isopropanol, und Ether, wie Diethylether oder Tetrahydrofuran. Die Reaktion

wird im allgemeinen bei 0 bis 30 °C, vorzugsweise bei 0 bis 20 °C durchgeführt. Hierzu setzt man auf 1 Mol des Ketones der Formel (II) etwa 1 Mol eines komplexen Hydrids, wie Natriumborhydrid oder Lithiumalanat, ein. Zur Isolierung der reduzierten Verbindungen der Formel (I) wird der Rückstand in verdünnter Salzsäure aufgenommen, anschließend alkalisch gestellt und mit einem organischen Lösungsmittel extrahiert. Die weitere Aufarbeitung erfolgt in üblicher Weise.

Arbeitet man mit Aluminiumisopropylat, so kommen als Verdünnungsmittel für die erfindungsmäße Umsetzung bevorzugt Alkohole, wie Isopropanol, oder inerte Kohlenwasserstoffe, wie Benzol, infrage. Die Reaktionstemperaturen können wiederum in einem größeren Bereich variiert werden ; im allgemeinen arbeitet man zwischen 20 und 120 °C, vorzugsweise bei 50 bis 100 °C. Zur Durchführung der Reaktion setzt man auf 1 Mol des Ketons der Formel (II) etwa 1 bis 2 Mol Aluminiumisopropylat ein. Zur Isolierung der reduzierten Verbindungen der Formel (I) wird das überschüssige Lösungsmittel durch Destillation im Vakuum entfernt und die entstandene Aluminiumverbindung mit verdünnter Schwefelsäure oder Natronlauge zersetzt. Die weitere Aufarbeitung erfolgt in üblicher Weise.

Arbeitet man mit Wasserstoff, so kommen als Verdünnungsmittel für die erfindungsgemäße Umsetzung polare organische Lösungsmittel infrage. Hierzu gehören vorzugsweise Alkohole, wie Methanol und Ethanol ; sowie Nitrile, wie Acetonitril. Die Umsetzung wird in Gegnwart eines Katalysators vorgenommen. Vorzugsweise werden Edelmetall-, Edelmetalloxid- bzw. Edelmetallhydroxid-Katalysatoren oder sogenannte « Raney-Katalysatoren » verwendet, insbesondere Platin, Platinoxid und Nickel. Die Reaktionstemperaturen können in einem größeren Bereich variiert werden. Im allgemeinen arbeitet man zwischen 20 und 50 °C. Die Reaktion kann bei Normaldruck, aber auch bei erhöhtem Druck, beispielsweise 1 bis 2 atü, durchgeführt werden. Zur Durchführung der Reaktion setzt man auf 1 Mol der Verbindung der Formel (II) etwa 1 Mol Wasserstoff und 0,1 Mol Katalysator ein. Zur Isolierung der reduzierten Verbindungen der Formel (I) wird vom Katalysator abfiltriert und vom Lösungsmittel im Vakuum befreit. Die weitere Aufarbeitung erfolgt in üblicher Weise.

Zur Herstellung von physiologisch verträglichen Säureadditionssalzen der Verbindungen der Formel (I) kommen vorzugsweise folgende Säuren infrage : Die Halogenwasserstoffsäuren, wie z. B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z. B. Essigsäure, Maleinsäure, Bernsteinsäure, Fumarsäure, Weinsäure, Zitronensäure, Salizylsäure, Sorbinsäure, Milchsäure, sowie Sulfonsäuren, wie z. B. p-Toluolsulfonsäure und 1,5-Naphthalindisulfonsäure.

Die Säureadditions-Salze der Verbindungen der Formel (I) können in einfacher Weise nach üblichen Salzbildungsmethoden, z. B. durch Lösen einer Verbindung der Formel (I) in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise Salze von Metallen der II. bis IV. Haupt- und der I. und II. sowie IV. bis VIII. Nebengruppe infrage, wobei Kupfer, Zink, Mangan, Magnesium, Zinn, Eisen und Nickel beispielhaft genannt seien.

Als Anionen der Salze kommen solche in Betracht, die sich vorzugsweise von folgenden Säuren ableiten : Halogenwasserstoffsäuren, wie z. B. die Chlorwasserstoffsäure und die Bromwasserstoffsäure, ferner Phosphorsäure, Salpetersäure und Schwefelsäure.

Die Metallsalz-Komplexe von Verbindungen der Formel (I) können in einfacher Weise nach üblichen Verfahren erhalten werden, so z. B. durch Lösen des Metallsalzes in Alkohol, z. B. Ethanol und Hinzufügen zur Verbindung der Formel (I). Man kann Metallsalz-Komplexe in bekannter Weise, z. B. durch Abfiltrieren, Isolieren und gegebenenfalls durch Umkristallisation reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Getreidekrankheiten, wie Getreidemehltau (Erysiphe graminis) und die Streifenkrankheit der Gerste ; von Erysiphe-Arten, wie den Erreger des Gurkenmehltaus (Erysiphe cichoracearum) ; von Fusicladium-Arten, wie den Erreger des Apfelschorfs (Fusicladium dendriticum) ; sowie von Reiskrankheiten, wie Pellicularia sasakii und Pyricularia oryzae, eingesetzt werden.

Die erfindungsgemäß verwendbaren Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen

und von der Art der Applikation. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park- und Sportanlagen, an Straßenrändern, auf Flughäfen oder in Obstanlagen reduziert werden. Von Bedeutung ist auch die Hemmung des Wuchses von krautigen und holzigen Pflanzen an Straßenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens (« Lagerns ») der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne daß die Gefahr besteht, daß das Getreide lagert.

Eine Hemmung des vegetativen Wachstums ermöglicht bei vielen Kulturpflanzen eine dichtere Anpflanzung, sodaß Mehrerträge bezogen auf die Bodenfläche erzielt werden können. Ein Vorteil der so erzielten kleineren Pflanzen ist auch, daß die Kultur leichter bearbeitet und beerntet werden kann.

Eine Hemmung des vegetativen Wachstums der Pflanzen kann auch dadurch zu Ertragsteigerungen führen, daß die Nährstoffe und Assimilate in stärkerem Maße der Blüten- und Fruchtbildung zugute kommen als den vegetativen Pflanzenteilen.

Mit Wachstumsregulatoren läßt sich häufig auch eine Forderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, dadurch daß mehr Assimilate gebildet werden, sodaß mehr oder größere Früchte entstehen.

Ertragssteigerungen können in manchen Fällen durch einen Eingriff in den pflanzlichen Stoffwechsel erreicht werden, ohne daß sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z. B. Zucker in Zuckerrüben oder Zuckerrohr, mit Wachstumsregulatoren vor oder nach der Ernte zu hemmen. Außerdem läßt sich die Produktion oder der Abfluß von sekundären Pflanzeninhaltsstoffen positiv beeinflussen. Als Beispiel sei die Stimulierung des Latexflusses bei Gummibäumen genannt.

Unter dem Einfluß von Wachstumsregulatoren kann es zur Ausbildung parthenokarper Früchte kommen. Ferner kann das Geschlecht der Blüten beeinflußt werden. Auch kann eine Sterilität des Pollens erzeugt werden, was bei der Züchtung und Herstellung von Hybridsaatgut eine große Bedeutung hat.

Durch den Einsatz von Wachstumsregulatoren läßt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung sehr erwünscht sein kann. Andererseits ist es aber auch möglich, das Wachstum der Seitentriebe zu hemmen. Für diese Wirkung besteht z. B. großes Interesse im Tabakanbau oder bei der Anpflanzung von Tomaten.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle, ist aber auch in anderen Kulturen wie z. B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso läßt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Maße gefördert werden (« Ausdünnung »), um die Alternanz zu brechen. Unter Alternanz versteht man die Eigenart einiger Obstarten, endogen bedingt von Jahr zu Jahr sehr unterschiedliche Erträge zu bringen. Schließlich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren läßt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüberhinaus kann mit Wachstumsregulatoren auch eine zeitliche Konzentrierung der Reife erzielt werden. Damit werden die Voraussetzungen dafür geschaffen, daß z. B. bei Tabak, Tomaten oder Kaffee eine vollständige mechanische oder manuelle Beerntung in einem Arbeitsgang vorgenommen werden kann.

Durch Anwendung von Wachstumsregulatoren kann ferner die Samen- oder Knospenruhe der

Pflanzen beeinflußt werden, sodaß die Pflanzen, wie z. B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes von Knospen oder der Keimung von Samen mit Hilfe von Wachstumsregulatoren kann in frostgefährdeten Gebieten erwünscht sein, um Schädigungen durch Spätfröste zu vermeiden.

Schließlich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z. B. durch vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage : Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgas, wie Halogenkohlenwasserstoffe sowie Butan, Propan, Stickstoff und Kohlendioxid. Als feste Trägerstoffe kommen in Frage : z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage : z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage : z. B. nichtionogene und anionische Emulgatoren, wie Polyoxyethylen-Fettsäureester, Polyoxyethylen-Fettalkoholether, z. B. Alkylarylpolyglycol-ether, Alkylsulfonate, Alkylsulfate, Arylsulfonate sowie Eiweißhydrolysate. Als Dispergiermittel kommen infrage : z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Allizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z. B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,000 1 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,000 01 bis 0,1 Gew.-%, vorzugsweise von 0,000 1 bis 0,02 %, am Wirkungsort erforderlich.

Bei der Verwendung als Pflanzenwachstumsregulatoren können die Wirkstoffkonzentrationen in einem größeren Bereich variiert werden. Im allgemeinen verwendet man pro Hektar Bodenfäche 0,01 bis 50 kg, bevorzugt 0,05 bis 10 kg an Wirkstoff.

Für die Anwendungszeit gilt, daß die Anwendung der Wachstumsregulatoren in einem bevorzugten Zeitraum vorgenommen wird, dessen genaue Abgrenzung sich nach den klimatischen und vegetativen Gegebenheiten richtet.

Herstellungsbeispiele

Beispiel 1

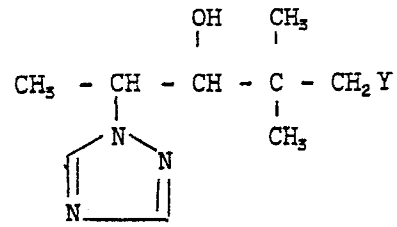

25,7 g (0,12 Mol) 1-Fluor-2,2-dimethyl-4-(1,2,4-triazol-1-yl)-pentan-3-on (erhalten aus dem Hydrochlorid durch Behandeln mit wässriger Natriumbicarbonatlösung) werden in 200 ml Methanol gelöst, 5 g (0,13 Mol) Natriumborhydrid portionsweise bei 10 °C zugegeben, 24 Stunden bei Raumtemperatur nachgerührt und 200 ml 2n Salzsäure zugetropft. Nach der Hydrolyse wird mit wässriger Natriumhydrogencarbonatlösung neutralisiert, zweimal mit je 200 ml Methylenchlorid extrahiert, die organische Phase mit 100 ml Wasser gewaschen, über Natriumsulfat getrocknet und das Lösungsmittel abdestilliert. Nach Umkristallisation aus einem Gemisch von 40 ml Ligroin und 30 ml Essigester erhält man 10,4 g (40 % der Theorie) 1-Fluor-2,2-dimethyl-4-(1,2,4-triazol-1-yl)-pentan-3-ol vom Schmelzpunkt 62-78 °C.

Herstellung des Ausgangsproduktes

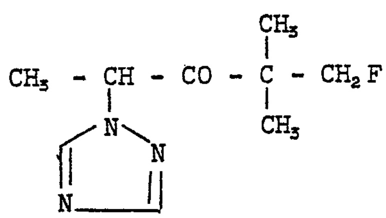 x HCl

(II - 1)

37,2 g (0,2 Mol) 1-Fluor-2,2-dimethyl-4-(1,2,4-triazol-1-yl)-butan-3-on werden in 200 ml Dimethylsulfoxid gelöst, 11,2 g (0,2 Mol) Kaliumhydroxid, in 24 ml Wasser gelöst, zugegeben und 28,4 g (0,2 Mol) Methyljodid unter Kühlung bei 20 °C eingetropft. Man läßt 24 Stunden bei Raumtemperatur nachrühren, gibt das Reaktionsgemisch auf 1 000 ml Wasser, extrahiert zweimal mit je 200 ml Methylenchlorid, wäscht die vereinigten organischen Phasen fünfmal mit je 100 ml Wasser, trocknet die organische Phase über Natriumsulfat, destilliert das Lösungsmittel ab, nimmt den Rückstand in 100 ml Aceton auf, filtriert und destilliert das Lösungsmittel der Mutterlauge ab. Der Rückstand wird in 150 ml Essigester aufgenommen und 14,4 g (0,02 Mol) Chlorwasserstoff eingeleitet. Danach läßt man auskristallisieren. Man erhält 33,8 g (72 % der Theorie) 1-Fluor-2,2-dimethyl-4-(1,2,4-triazol-1-yl)-pentan-3-on-hydrochlorid vom Schmelzpunkt 142 °C.

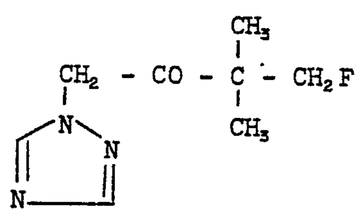 (I)

4,18 kg (35,4 Mol) 1-Fluor-2,2-dimethyl-butan-3-on werden in 30 l Methylenchlorid gelöst, 5,67 kg Brom bei 20 °C innerhalb von 2 Stunden so zugetropft, daß laufend Entfärbung eintritt. Das Lösungsmittel wird im Wasserstrahlvakuum abdestilliert, nochmals 15 l Methylenchlorid zum Rückstand gegeben und erneut das Lösungsmittel im Wasserstrahlvakuum abdestilliert. Das rohe 1-Fluor-4-brom-2,2-dimethyl-butan-3-on (6,97 kg quantitative Ausbeute) wird zu 2,45 kg 1,2,4-Triazol, 4,89 kg Kaliumcarbonat in 21,4 l Aceton bei 30 bis 35 °C unter Kühlung innerhalb von 2 Stunden zugetropft. Man läßt 15 Stunden bei Raumtemperatur nachrühren, filtriert vom Ungelösten ab und destilliert das Lösungsmittel im Wasserstrahlvakuum ab. Man erhält 6,12 kg (93 % der Theorie) 1-Fluor-2,2-dimethyl-4-(1,2,4-triazol-1-yl)-butan-3-on, das direkt weiter umgesetzt werden kann.

In entsprechender Weise werden die nachfolgenden erfindungsgemäßen Verbindungen der allgemeinen Formel

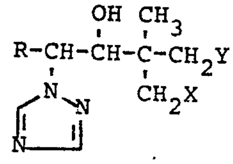 (I)

erhalten :

| Bsp.Nr. | R | X | Y | Schmelzpunkt (°C) |
|---|---|---|---|---|
| 2 | 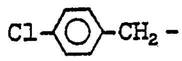 | H | F | 92-106 |
| 3 | 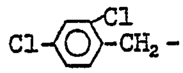 | H | F | 116-21 |
| 4 | 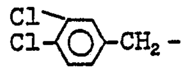 | H | F | 158-74 |
| 5 | 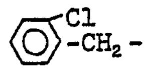 | H | F | 108-12 |
| 6 | 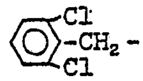 | H | F | 116-22 |
| 7 | 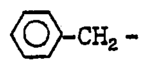 | H | F | 78-88 |
| 8 | 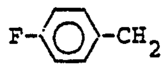 | H | F | 76-84 |
| 9 | 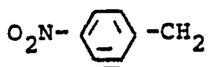 | H | F | 143-54 (Zers.) |
| 10 | 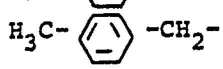 | H | F | 62-72 |
| 11 | $C_4H_9$- | H | F | $n_D^{20}$=1,4738 |
| 12 | 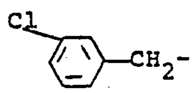 | H | F | 108-28 (Zers.) |
| 13 | $H_2C$=CH-$CH_2$- | H | F | Öl |
| 14 | $(H_3C)_2$CH- | H | F | 161-69 |
| 15 | 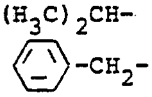 | F | F | 92-111 |
| 16 | HC≡C-$CH_2$- | H | F | 82-88 |
| 17 | 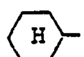 | H | F | 40 |
| 18 | 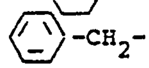 | H | Cl | 82-88 |
| 19 | $H_2C$=CH-$CH_2$-$CH_2$- | H | F | Öl |
| 20 | 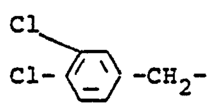 | F | F | 156-68 (Zers.) |
| 21 | 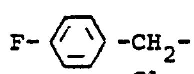 | H | Cl | 98-104 |
| 22 | 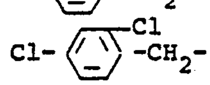 | F | F | 108-26 (Zers.) |
| 23 | 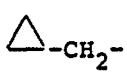 | H | F | 76-88 |
| 24 | 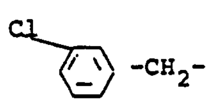 | H | Cl | 108-12 |
| 25 | 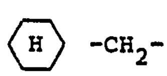 | H | F | Öl |

(Fortsetzung)

| Beispiel Nr. | R | X | Y | Schmelzpunkt (°C) |
|---|---|---|---|---|
| 26 | $-C_2H_5$ | H | F | Öl |
| 27 | 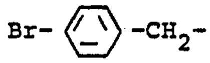 | H | F | 141-44 |
| 28 | 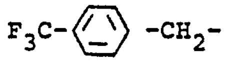 | H | F | 73-76 |
| 29 | 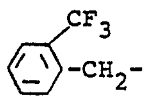 | H | F | 86-92 |
| 30 | 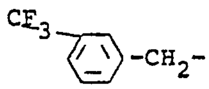 | H | F | 63-8 |
| 31 | 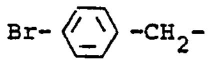 | H | Cl | 108-21 |
| 32 | 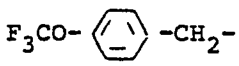 | H | F | 68-72 |
| 33 | 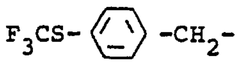 | H | F | 86-90 |
| 34 | 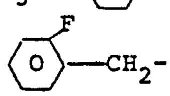 | H | F | 88-98 |
| 35 | 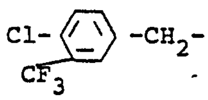 | H | F | 72-112 |
| 36 | 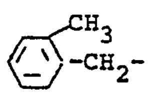 | H | F | 112-23 |
| 37 | 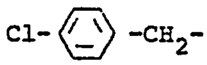 | H | F | semikristallin |
| 38 | 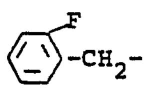 | Cl | Cl | semikristallin |
| 39 | 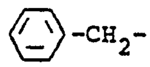 | Cl | Cl | 136-7 |
| 40 | 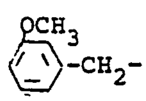 | H | F | 90-108 |
| 41 | 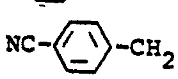 | H | F | 143-6 |

Die folgenden Ausgangsstoffe der Formel

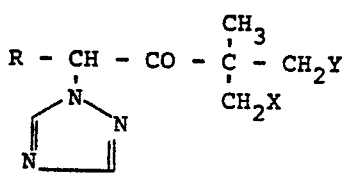

(II)

werden nach der im Beispiel 1 beschriebenen Methode erhalten.

| Bsp. Nr. | R | X | Y | Schmelzpunkt (°C) |
|---|---|---|---|---|
| II-2 | 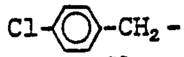 | H | F | 78-79 |
| II-3 | 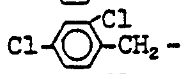 | H | F | 112-20 |
| II-4 | 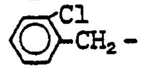 | H | F | 62-72 |
| II-5 | 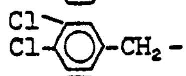 | H | F | 58-70 |
| II-6 | 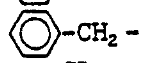 | H | F | 150(Zers.)(xHCl) |
| II-7 | 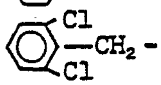 | H | F | 80-92 |
| II-8 | 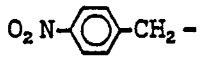 | H | F | 138-40 |
| II-9 | i-$C_3H_7$- | H | F | 45(xHCl) |
| II-10 | 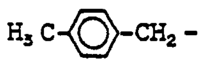 | H | F | 94 |
| II-11 | $C_2H_5$ | H | F | $Kp_{0,05}$ 152 |
| II-12 | 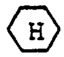 | H | F | 128 |
| II-13 | $C_4H_9$ | H | F | $Kp_{0,05}$ 163 |
| II-14 | $CH_2=CH-CH_2-$ | H | F | Oel |
| II-15 | $CH\equiv C-CH_2-$ | H | F | 130 (Zers.) (xHCl) |
| II-16 | 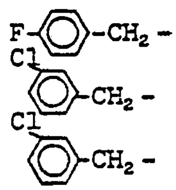 | H | F | 182(Zers.)(xHCl) |
| II-17 | | H | F | 99 |
| II-18 | | H | Cl | 102 |
| II-19 | 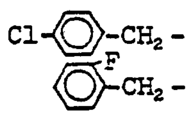 | F | F | 108 |
| II-20 | | Cl | Cl | Oel |
| II-21 | 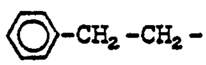 | H | F | Oel |
| II-22 | 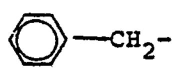 | F | F | Oel |
| II-23 | 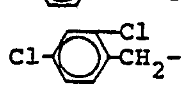 | F | F | 63-78 |
| II-24 | 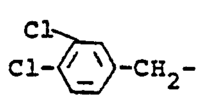 | F | F | 96-112 (xHCl) (Zersetz.) |
| II-25 | 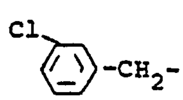 | H | Cl | 116-27 (xHCl) |
| II-26 | 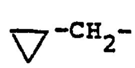 | H | F | 107 (xHCl) (Zersetz.) |
| II-27 | 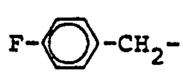 | H | Cl | 58-78 |
| II-28 | 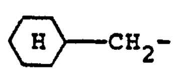 | H | F | 88-98 (xHCl) |

(Fortsetzung)

| Bsp. Nr. | R | X | Y | Schmelzpunkt (°C) |
|---|---|---|---|---|
| II-29 | 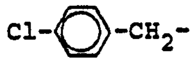 | H | Cl | 58-74 |
| II-30 | $CH_2=CH-CH_2-CH_2-$ | H | F | Oel |
| II-31 | 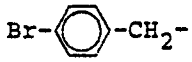 | H | F | 78 |
| II-32 | 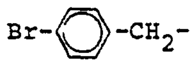 | H | Cl | 56 |
| II-33 | 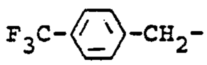 | H | F | 82 |
| II-34 | 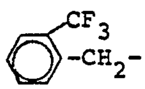 | H | F | 86 |
| II-35 | 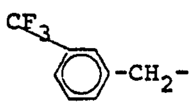 | H | F | 88 |
| II-36 | 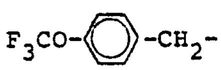 | H | F | 86-88 |
| II-37 | 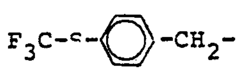 | H | F | 86-92 |
| II-38 | 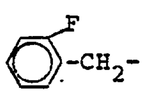 | H | F | 46-48 |
| II-39 | 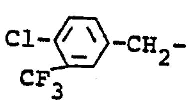 | H | F | 53-63 |
| II-40 | 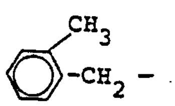 | H | F | 62-4 |
| II-41 | 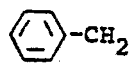 | Cl | Cl | Öl $n_D^{20}$ 1,5408 |
| II-42 | 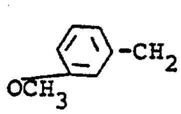 | H | F | 78-81 |
| II-43 | 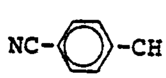 | H | F | 138-41 |

## Anwendungsbeispiele

Bei der Prüfung auf fungizide Wirksamkeit werden in den nachfolgenden Beispielen die nachstehend angegebenen Verbindungen als Vergleichssubstanzen eingesetzt :

A = 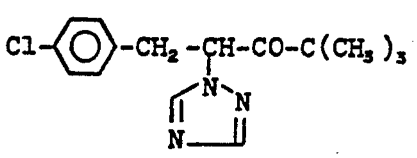 (bekannt aus DE-A 27 34 426)

B = 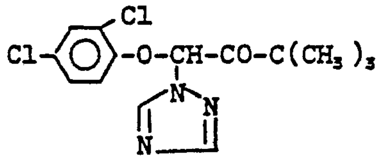 (bekannt aus DE-B 22 01 063)

C = 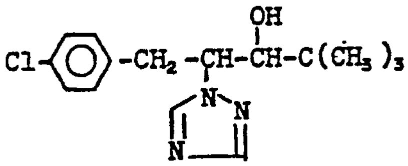 (bekannt aus DE-A 27 37 489 und DE-A 27 34 426)

Beispiel A

Sproßbehandlungs-Test/Getreidemehltau/Protektiv (blattzerstörende Mykose)

Zur Herstellung einer zweckmäßigen Wirkstoffzubereitung nimmt man 0,25 Gewichtsteile Wirkstoff in 25 Gewichtsteilen Dimethylformamid und 0,06 Gewichtsteilen Emulgator (Alkyl-aryl-polyglykolether) auf und gibt 975 Gewichtsteile Wasser hinzu. Das Konzentrat verdünnt man mit Wasser auf die gewünschte Endkonzentration der Spritzbrühe.

Zur Prüfung auf protektive Wirksamkeit besprüht man die einblättrigen Gerstenjungpflanzen der Sorte Amsel mit der Wirkstoffzubereitung taufeucht. Nach Antrocknen bestäubt man die Gerstenpflanzen mit Sporen von Erysiphe graminis var. hordei.

Nach 6 Tagen Verweilzeit der Pflanzen bei einer Temperatur von 21-22 °C und einer Luftfeuchtigkeit von 80-90 % wertet man den Besatz der Pflanzen mit Mehltaupusteln aus. Der Befallsgrad wird in Prozent des Befalls der unbehandelten Kontrollpflanzen ausgedrückt. Dabei bedeutet 0 % keinen Befall und 100 % den gleichen Befallsgrad wie bei der unbehandelten Kontrolle. Der Wirkstoff ist umso wirksamer, je geringer der Mehltaubefall ist.

Bei diesem Test zeigen z. B. die folgenden Verbindungen eine sehr gute Wirkung, die derjenigen der aus dem Stand der Technik bekannten Verbindung (A) überlegen ist : Verbindungen gemäß Herstellungsbeispielen 2,3,4,5 und 6.

Tabelle A

Sproßbehandlungs-Test/Getreidemehltau/Protektiv

| Wirkstoffe | Wirkstoffkonzentration in der Spritzbrühe in Gew.% | Befall in % der unbehandelten kontrolle |
|---|---|---|
| 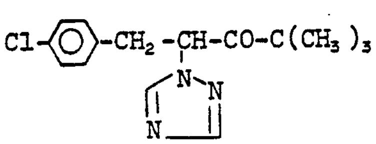 (bekannt) (A) | 0,001 | 72,5 |
| 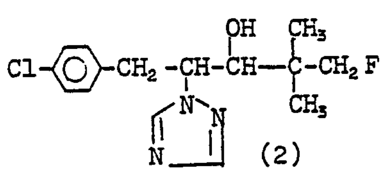 (2) | 0,001 | 0,0 |
| 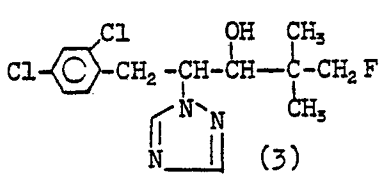 (3) | 0,001 | 0,0 |

Tabelle A (Fortsetzung)

Sproßbehandlungs-Test/ Getreidemehltau/ Protektiv

| W i r k s t o f f e | Wirkstoff-konzentration in der Spritzbrühe in Gew.% | Befall in % der unbehandelten kontrolle |
|---|---|---|
| 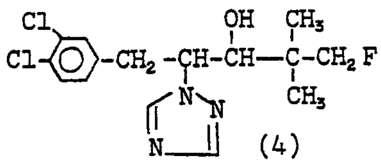 (4) | 0,001 | 3,8 |
| 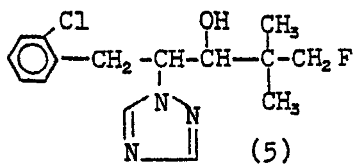 (5) | 0,001 | 50,0 |
| 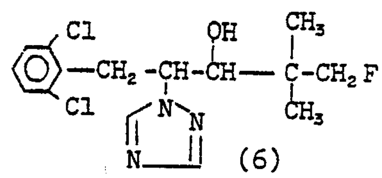 (6) | 0,001 | 33,8 |

Beispiel B

Saatgutbeizmittel-Test/Streifenkrankheit der Gerste (samenbürtige Mykose)

Zur Herstellung eines zweckmäßigen Trockenbeizmittels verstreckt man den Wirkstoff mit einem Gemisch aus gleichen Gewichtsteilen Talkum und Kieselgur zu einer feinpulverigen Mischung mit der gewünschten Wirkstoffkonzentration. Zur Beizung schüttelt man Gerstensaatgut, das durch Drechslera graminea (Syn. Helminthosporium gramineum) natürlich verseucht ist, mit dem Beizmittel in einer verschlossenen Glasflasche. Das Saatgut setzt man auf feuchten Filterscheiben in verschlossenen Petrischalen im Kühlschrank 10 Tage lang einer Temperatur von 4 °C aus. Dabei wird die Keimung der Gerste und gegebenenfalls auch der Pilzsporen eingeleitet. Anschließend sät man die vorgekeimte Gerste mit 2 × 50 Korn 3 cm tief in Frühstorfer Einheitserde und kultiviert sie im Gewächshaus bei Temperaturen um 18 °C in Saatkästen, die täglich 16 Stunden dem Licht ausgesetzt werden. Innerhalb von 3 bis 4 Wochen bilden sich die typischen Symptome der Streifenkrankheit aus.

Nach dieser Zeit bestimmt man die Anzahl der kranken Pflanzen in Prozent der insgesamt aufgelaufenen Pflanzen. Der Wirkstoff ist umso wirksamer je weniger Pflanzen erkrankt sind.

Bei diesem Test zeigen z. B. die folgenden Verbindungen eine sehr gute Wirkung, die derjenigen der aus dem Stand der Technik bekannten Verbindung (C) überlegen ist : Verbindung gemäß Herstellungsbeispiel 2.

Tabelle B

Saatgutbeizmittel-Test/Streifenkrankheit der Gerste

| W i r k s t o f f | Wirkstoffkonzentration im Beizmittel in % | Beizmittelaufwandmenge in g/kg Saatgut | Anzahl streifenkranker Pflanzen in % der insgesamt aufgelaufenen Pflanzen |
|---|---|---|---|
| ungebeizt | - | - | 33,8 |

Tabelle B (Fortsetzung)

| Wirkstoff | Wirkstoffkonzentration im Beizmittel in % | Beizmittelaufwandmenge in g/kg Saatgut | Anzahl streifenkranker Pflanzen in % der insgesamt aufgelaufenen Pflanzen |
|---|---|---|---|
| 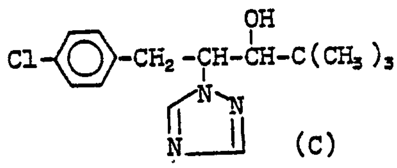 (C) (bekannt) | 25 | 2 | 3,2 |
| 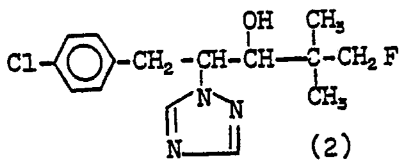 (2) | 25 | 2 | 0,0 |

Beispiel C

Fusicladium-Test (Apfel)/protektiv

Lösungsmittel : 4,7 Gewichtsteile Aceton
Emulgator : 0,3 Gewichtsteile Alkylarylpolyglykolether
Wasser : 95,0 Gewichtsteile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Apfelsämlinge, die sich im 4- bis 6-Blattstadium befinden, bis zur Tropfnässe. Die Pflanzen verbleiben 24 Stunden bei 20 °C und einer relativen Luftfeuchtigkeit von 70 % im Gewächshaus. Anschließend werden sie mit einer wässrigen Konidiensuspension des Apfelschorferregers (Fusicladium dendriticum) inokuliert und 18 Stunden lang in einer Feuchtkammer bei 18 bis 20 °C und 100 % relativer Luftfeuchtigkeit inkubiert.

Die Pflanzen kommen dann erneut für 14 Tage ins Gewächshaus.

15 Tage nach der Inokulation wird der Befall der Sämlinge bestimmt. Die erhaltenen Boniturwerte werden in Prozent Befall umgerechnet. 0 % bedeutet keinen Befall, 100 % bedeudet, daß die Pflanzen vollständig befallen sind.

Bei diesem Test zeigen z. B. die folgenden Verbindungen eine sehr gute unWirkg, die derjenigen der aus dem Stand der Technik bekannten Verbindung (B) überlegen ist : Verbindung gemäß Herstellungsbeispiel 2.

Tabelle C

Fusicladium-Test (Apfel)/Protektiv

| Wirkstoff | Befall in % bei einer Wirkstoffkonzentration von 0,00025 % |
|---|---|
| 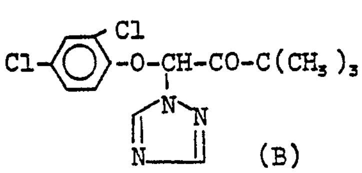 (B) (bekannt) | 46 |

Tabelle C (Fortsetzung)

| Wirkstoff | Befall in % bei einer Wirkstoffkonzentration von 0,00025 % |
|---|---|
| 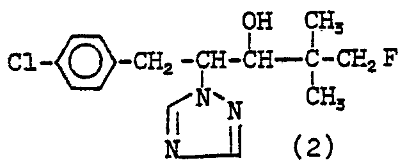 (2) | 14 |

Beispiel D

Erysiphe-Test (Gurken)/Protektiv

Lösungsmittel : 4,7 Gewichtsteile Aceton
Emulgator : 0,3 Gewichtsteile Alkyl-aryl-polyglykolether
Wasser : 95,0 Gewichtsteile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Gurkenpflanzen mit etwa drei Laubblättern bis zur Tropfnässe. Die Gurkenpflanzen verbleiben zur Trocknung 24 Stunden im Gewächshaus. Dann werden sie zur Inokulation mit Konidien des Pilzes Erysiphe cichoreacearum bestäubt. Die Pflanzen werden anschließend bei 23 bis 24 °C und bei einer relativen Luftfeuchtigkeit von ca 75 % im Gewächshaus aufgestellt.

Nach 12 Stunden wird der Befall der Gurkenpflanzen bestimmt. Die erhaltenen Boniturwerte werden in Prozent Befall umgerechnet. 0 % bedeudet keinen Befall, 100 % bedeutet, daß die Pflanzen vollständig befallen sind.

Bei diesem Test zeigen z. B. die folgenden Verbindungen eine sehr gute Wirkung, die derjenigen der aus dem Stand der Technik bekannten Verbindung (A) überlegen ist : Verbindung gemäß Herstellungsbeispiel 2.

Tabelle D
Erysiphe-Test (Gurken)/Protektiv

| Wirkstoff | Befall in % bei einer Wirkstoffkonzentration von 0,0001 % |
|---|---|
| 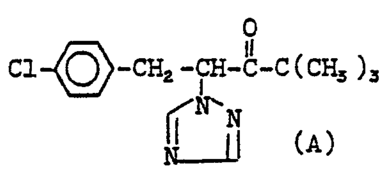 (A) (bekannt) | 84 |
| 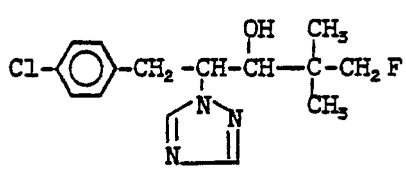 (2) | 19 |

Beispiel E

Wuchsbeeinflussung bei Zuckerrüben

Lösungsmittel : 30 Gewichtsteile Dimethylformamid
Emulgator : 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Zuckerrüben werden im Gewächshaus bis zur vollen Ausbildung der Keimblätter angezogen. In diesem Stadium werden die Pflanzen tropfnaß mit den Wirkstoffzubereitung besprüht. Nach 14 Tagen wird der Zuwachs der Pflanzen gemessen und die Wuchsbeeinflussung in Prozent des Zuwachses der Kontrollpflanzen berechnet. Es bedeuten 0 % Wuchsbeeinflussung ein Wachstum entsprechend dem der Kontrollpflanzen. Negative Werte kennzeichnen eine Wuchshemmung, positive Werte eine Wuchsförderung gegenüber den Kontrollpflanzen.

Die erfindungsgemäßen Wirkstoffe 2, 3 und 4 zeigen in diesem Test eine starke Wuchsbeeinflussung gegenüber der Kontrolle.

Tabelle E
Wuchsbeeinflussung bei Zuckerrüben

| Wirkstoffe | Konzentration in % | Wuchsbeeinflussung in % |
|---|---|---|
| 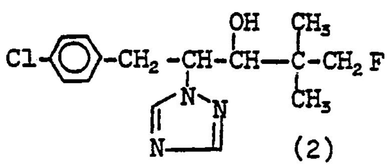 (2) | 0,05 | -45 *) **) |
| 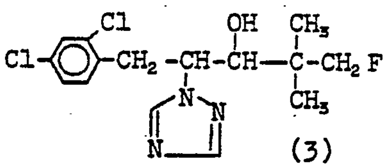 (3) | 0,05 | -5 *) **) |
| 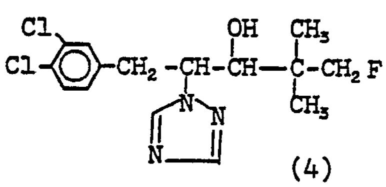 (4) | 0,05 | +5 |
| Kontrolle | - | = 0 |

*) dunkelgrün
**) dicke Blätter

## Beispiel F

Wuchshemmung bei Gerste

Lösungsmittel : 30 Gewichtsteile Dimethylformamid
Emulgator : 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Gerstenpflanzen werden im Gewächshaus bis zum 2-Blattstadium angezogen. In diesem Stadium werden die Pflanzen tropfnaß mit den Wirkstoffzubereitungen besprüht. Nach 3 Wochen wird bei allen Pflanzen der Zuwachs gemessen und die Wuchshemmung in Prozent des Zuwachses der Kontrollpflanzen berechnet. Es bedeuten 100 % Wuchshemmung den Stillstand des Wachstums und 0 % ein Wachstum entsprechend der Kontrollpflanzen.

Der erfindungsgemäße Wirkstoff 2 zeigt in diesem Test gegenüber der Kontrolle eine starke Wuchshemmung.

Tabelle F

| Wirkstoff | Konzentration in % | Wuchshemmung in % |
|---|---|---|
| 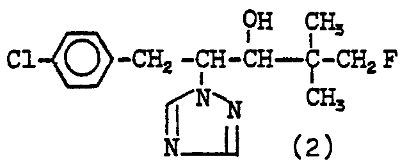 (2) | 0,05 | 35 |
| Kontrolle | - | = 0 |

## Beispiel G

Wuchshemmung bei Gras (Festuca pratensis)

Lösungsmittel : 30 Gewichtsteile Dimethylformamid
Emulgator : 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Gras (Festuca pratensis) wird im Gewächshaus bis zu einer Wuchshöhe von 5 cm angezogen. In diesem Stadium werden die Pflanzen mit den Wirkstoffzubereitungen tropfnass besprüht. Nach 3 Wochen wird der Zuwachs gemessen und die Wuchshemmung in Prozent des Zuwachses der Kontrollpflanzen berechnet. Es bedeuten 100 % Wuchshemmung den Stillstand des Wachstumes und 0 % ein Wachstum entsprechend dem der Kontrollpflanzen.

Der erfindungsgemäße Wirkstoff 2 zeigt in diesem Test gegenüber der Kontrolle eine starke Wuchshemmung.

Tabelle G

Wuchshemmung bei Gras (Festuca pratensis)

| Wirkstoff | Konzentration in % | Wuchshemmung in % |
|---|---|---|
| 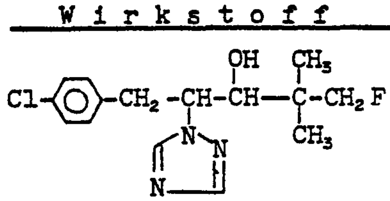 (2) | 0,05 | 80 |
| Kontrolle | - | = 0 |

Beispiel H

Wuchshemmung bei Sojabohnen

Lösungsmittel : 30 Gewichtsteile Dimethylformamid
Emulgator : 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Sojabohnenpflanzen werden im Gewächshaus bis zur vollen Entfaltung des ersten Folgeblattes angezogen. In diesem Stadium werden die Pflanzen tropfnaß mit den Wirkstoffzubereitungen besprüht. Nach 3 Wochen wird bei allen Pflanzen der Zuwachs gemessen und die Wuchshemmung in Prozent des Zuwachses der Kontrollpflanzen berechnet. Es bedeuten 100 % Wuchshemmung den Stillstand des Wachstums und 0 % ein Wachstum entsprechend dem der Kontrollpflanzen.

Die erfindungsgemäßen Wirkstoffe 2, 3 und 4 zeigen in diesem Test gegenüber der Kontrolle eine starke Wuchshemmung.

Tabelle H

Wuchshemmung bei Sojabohnen

| Wirkstoffe | Wirkstoffkonzentration in % | Wuchshemmung in % |
|---|---|---|
| 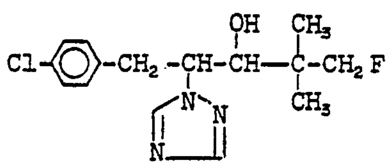 (2) | 0,05 | 90 *) |
| 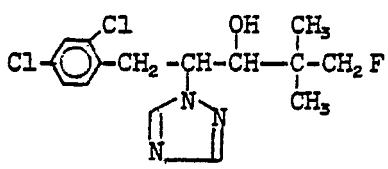 (3) | 0,05 | 85 *) |
| 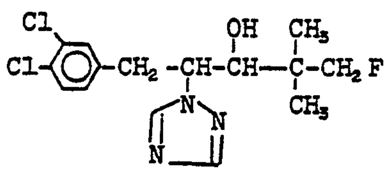 (4) | 0,05 | 95 *) |
| Kontrolle | - | = 0 |

*) dunkelgrüne Blattfarbe

Beispiel I

Wuchshemmung bei Baumwolle

Lösungsmittel : 30 Gewichtsteile Dimethylformamid
Emulgator : 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit der angegebenen Menge Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 3 Wochen wird der Zuwachs der Pflanzen gemessen und die Wuchshemmung in Prozent des Zuwachses der Kontrollpflanzen berechnet. Es bedeuten 100 % Wuchshemmung den Stillstand des Wachstums und 0 % ein Wachstum entsprechend dem der Kontrollpflanzen.

Der erfindungsgemäße Wirkstoff 2 zeigt in diesem Test gegenüber der Kontrolle eine starke Wuchshemmung.

Tabelle I

Wuchshemmung bei Baumwolle

| W i r k s t o f f e | Konzentration in % | Wuchshemmung in % |
|---|---|---|
| 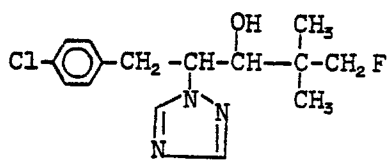 (2) | 0,05 | 90 *) |
| Kontrolle | - | = 0 |

*) dunkelgrüne Blattfarbe

**Ansprüche**

1. Substituierte Triazolylmethyl-tert.-butyl-carbinole der Formel

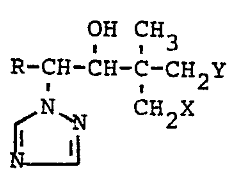 (I)

in welcher

R für Alkyl mit 1 bis 12 Kohlenstoffatomen, Alkenyl mit 2 bis 12 Kohlenstoffatomen, Alkinyl mit 2 bis 12 Kohlenstoffatomen, gegebenenfalls durch Alkyl mit 1 bis 4 Kohlenstoffatomen substituiertes Cycloalkyl mit 3 bis 7 Kohlenstoffatomen, gegebenenfalls durch Alkyl mit 1 bis 4 Kohlenstoffatomen

substituiertes Cycloalkylalkyl mit 3 bis 7 Kohlenstoffatomen im Cycloalkylteil und 1 bis 4 Kohlenstoffatomen im Alkylteil sowie für Aralkyl mit 6 bis 10 Kohlenstoffatomen im Arylteil und 1 bis 4 Kohlenstoffatomen im Alkylteil steht, wobei der Arylteil substituiert sein kann durch Alkyl mit 1 bis 4 Kohlenstoffatomen, Alkoxy mit 1 bis 4 Kohlenstoffatomen, Alkylthio mit 1 bis 4 Kohlenstoffatomen, Halogen, Halogenalkyl mit 1 bis 4 Kohlenstoffatomen und 1 bis 5 gleichen oder verschiedenen Halogenatomen, Halogenalkoxy mit 1 bis 4 Kohlenstoffatomen und 1 bis 5 gleichen oder verschiedenen Halogenatomen, Halogenalkylthio mit 1 bis 4 Kohlenstoffatomen und 1 bis 5 gleichen oder verschiedenen Halogenatomen, Nitro, Cyano, Alkoxycarbonyl mit 1 bis 4 Kohlenstoffatomen im Alkoxyteil, Amino, Alkylamino mit 1 bis 4 Kohlenstoffatomen, Dialkylamino mit 1 bis 4 Kohlenstoffatomen in jedem Alkylteil, Phenylamino, Phenoxy und/oder Phenyl, welches seinerseits substituiert sein kann durch Halogen und/oder Alkyl mit 1 bis 2 Kohlenstoffatomen,

X für Wasserstoff steht und

Y für Fluor oder Chlor steht oder

X und Y gleich sind und für Fluor oder Chlor stehen,

sowie deren pflanzenverträgliche Säureadditions-Salze und Metallsalz-Komplexe.

2. Verfahren zur Herstellung von substituierten Triazolylmethyl-tert.-butyl-carbinolen der Formel

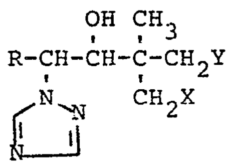

(I)

in welcher

R für Alkyl mit 1 bis 12 Kohlenstoffatomen, Alkenyl mit 2 bis 12 Kohlenstoffatomen, Alkinyl mit 2 bis 12 Kohlenstoffatomen, gegebenenfalls durch Alkyl mit 1 bis 4 Kohlenstoffatomen substituiertes Cycloalkyl mit 3 bis 7 Kohlenstoffatomen, gegebenenfalls durch Alkyl mit 1 bis 4 Kohlenstoffatomen substituiertes Cycloalkylalkyl mit 3 bis 7 Kohlenstoffatomen im Cycloalkylteil und 1 bis 4 Kohlenstoffatomen im Alkylteil sowie für Aralkyl mit 6 bis 10 Kohlenstoffatomen im Arylteil und 1 bis 4 Kohlenstoffatomen im Alkylteil steht, wobei der Arylteil substituiert sein kann durch Alkyl mit 1 bis 4 Kohlenstoffatomen, Alkoxy mit 1 bis 4 Kohlenstoffatomen, Alkylthio mit 1 bis 4 Kohlenstoffatomen, Halogen, Halogenalkyl mit 1 bis 4 Kohlenstoffatomen und 1 bis 5 gleichen oder verschiedenen Halogenatomen, Halogenalkoxy mit 1 bis 4 Kohlenstoffatomen und 1 bis 5 gleichen oder verschiedenen Halogenatomen, Halogenalkylthio mit 1 bis 4 Kohlenstoffatomen und 1 bis 5 gleichen oder verschiedenen Halogenatomen, Nitro, Cyano, Alkoxycarbonyl mit 1 bis 4 Kohlenstoffatomen im Alkoxyteil, Amino, Alkylamino mit 1 bis 4 Kohlenstoffatomen, Dialkylamino mit 1 bis 4 Kohlenstoffatomen in jedem Alkylteil, Phenylamino, Phenoxy und/oder Phenyl, welches seinerseits substituiert sein kann durch Halogen und/oder Alkyl mit 1 bis 2 Kohlenstoffatomen,

X für Wasserstoff steht und

Y für Fluor oder Chlor steht oder

X und Y gleich sind und für Fluor oder Chlor stehen,

sowie von deren pflanzenverträglichen Säureadditions-Salzen und Metallsalz-Komplexen, dadurch gekennzeichnet, daß man substituierte Triazolyl-methyl-tert.-butyl-ketone der Formel

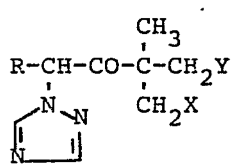

(II)

in welcher R, X und Y die oben angegebene Bedeutung haben, nach bekannten Methoden in üblicher Weise reduziert und gegebenenfalls anschließend eine Säure oder ein Metallsalz addiert.

3. Pflanzenschutzmittel, gekennzeichnet durch einen Gehalt an mindestens einem substituierten Triazolylmethyl-tert.-butyl-carbinol der Formel (I) bzw. einem Säureadditions-Salz oder Metallsalz-Komplex eines Stoffes der Formel (I).

4. Verfahren zur Bekämpfung von Pilzen, dadurch gekennzeichnet, daß man substituierte Triazolylmethyl-tert.-butyl-carbinole der Formel (I) bzw. Säureadditions-Salze oder Metallsalz-Komplexe von Stoffen der Formel (I) auf Pilze oder ihren Lebensraum einwirken läßt.

5. Verfahren zur Regulierung des Pflanzenwachstums, dadurch gekennzeichnet, daß man substituierte Triazolylmethyl-tert.-butyl-carbinole der Formel (I) bzw. Säureadditions-Salze oder Metallsalz-Komplexe von Stoffen der Formel (I) auf die zu behandelnden Pflanzen oder ihren Lebensraum einwirken läßt.

6. Verwendung von substituierten Triazolylmethyl-tert.-butyl-carbinolen der Formel (I) bzw. von Säureadditions-Salzen oder Metallsalz-Komplexen von Stoffen der Formel (I) als Pflanzenschutzmittel.

7. Verwendung von substituierten Triazolylmethyl-tert.-butyl-carbinolen der Formel (I) bzw. von Säureadditions-Salzen oder Metallsalz-Komplexen von Stoffen der Formel (I) zur Bekämpfung von Pilzen.

8. Verwendung von substituierten Triazolylmethyl-tert.-butyl-carbinolen der Formel (I) bzw. von Säureadditions-Salzen oder Metallsalz-Komplexen von Stoffen der Formel (I) zur Regulierung des Pflanzenwachstums.

9. Verwendung von substituierten Triazolylmethyl-tert.-butyl-carbinolen der Formel (I) bzw. von Säureadditions-Salzen oder Metallsalz-Komplexen von Stoffen der Formel (I) als Zwischenprodukte zur Herstellung von Pflanzenschutzmitteln.

10. Verfahren zur Herstellung von Pflanzenschutzmitteln, dadurch gekennzeichnet, daß man substituierte Triazolylmethyl-tert.-butyl-carbinole der Formel (I) bzw. Säureadditions-Salze oder Metallsalz-Komplexe von Stoffen der Formel (I) mit Streckmitteln und/oder oberflächenaktiven Stoffen vermischt.

## Claims

1. Substituted triazolylmethyl-tert.-butyl-carbinols of the formula

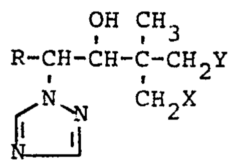

(I)

in which

R represents alkyl with 1 to 12 carbons atoms, alkenyl with 2 to 12 carbon atoms, alkinyl with 2 to 12 carbon atoms, cycloalkyl with 3 to 7 carbon atoms which is optionally substituted by alkyl with 1 to 4 carbons atoms, cycloalkylalkyl which has 3 to 7 carbon atoms in the cycloalkyl part and 1 to 4 carbon atoms in the alkyl part and is optionally substituted by alkyl with 1 to 4 carbon atoms, or aralkyl with 6 to 10 carbon atoms in the aryl part and 1 to 4 carbon atoms in the alkyl part, it being possible for the aryl part to be substituted by alkyl with 1 to 4 carbon atoms, alkoxy with 1 to 4 carbon atoms, alkylthio with 1 to 4 carbon atoms, halogen, halogenoalkyl with 1 to 4 carbon atoms and 1 to 5 identical or different halogen atoms, halogenoalkoxy with 1 to 4 carbon atoms and 1 to 5 identical or different halogen atoms, halogenoalkylthio with 1 to 4 carbon atoms and 1 to 5 identical or different halogen atoms, nitro, cyano, alkoxycarbonyl with 1 to 4 carbon atoms in the alkoxy part, amino, alkylamino with 1 to 4 carbon atoms, dialkylamino with 1 to 4 carbon atoms in each alkyl part, phenylamino, phenoxy and/or phenyl, which in turn can be substituted by halogen and/or alkyl with 1 to 2 carbon atoms,

X represents hydrogen and

Y represents fluorine or chlorine or

X and Y are identical and represent fluorine or chlorine,

and acid addition salts and metal salt complexes thereof which are tolerated by plants.

2. Process for the preparation of substituted triazolyl-methyl-tert.-butyl-carbinols of the formula

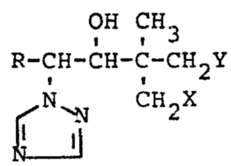

(I)

in which

R represents alkyl with 1 to 12 carbon atoms, alkenyl with 2 to 12 carbon atoms, alkinyl with 2 to 12 carbon atoms, cycloalkyl with 3 to 7 carbon atoms which is optionally substituted by alkyl with 1 to 4 carbon atoms, cycloalkylalkyl which has 3 to 7 carbon atoms in the cycloalkyl part and 1 to 4 carbon atoms in the alkyl part and is optionally substituted by alkyl with 1 to 4 carbon atoms, or aralkyl with 6 to 10 carbon atoms in the aryl part and 1 to 4 carbon atoms in the alkyl part, it being possible for the aryl part to be substituted by alkyl with 1 to 4 carbon atoms, alkoxy with 1 to 4 carbon atoms, alkylthio with 1 to 4 carbon atoms, halogen, halogenoalkyl with 1 to 4 carbon atoms and 1 to 5 identical or different halogen atoms, halogenoalkoxy with 1 to 4 carbon atoms and 1 to 5 identical or different halogen atoms, halogenoalkylthio with 1 to 4 carbon atoms and 1 to 5 identical or different halogen atoms, nitro, cyano, alkoxycarbonyl with 1 to 4 carbon atoms in the alkoxy part, amino, alkylamino with 1 to 4 carbon atoms, dialkylamino with 1 to 4 carbon atoms in each alkyl part, phenylamino, phenoxy and/or phenyl, which in turn can be substituted by halogen and/or alkyl with 1 to 2 carbon atoms,

X represents hydrogen and

Y represents fluorine or chlorine or

X and Y are identical and represent fluorine or chlorine,

and acid addition salts and metal salt complexes thereof which are tolerated by plants, characterised in that substituted triazolylmethyl-tert.-butyl ketones of the formula

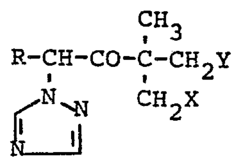

(II)

in which R, X and Y have the meaning indicated above, are reduced in the customary manner by known methods and, if desired, an acid or a metal salt is then added on.

3. Plant protection agents, characterised in that they contain at least one substituted triazolylmethyl-tert.-butyl-carbinol of the formula (I) or an acid addition salt or metal salt complex of a substance of the formula (I).

4. Process for combating fungi, characterised in that substituted triazolylmethyl-tert.-butyl-carbinols of the formula (I) or acid addition salts or metal salt complexes of substances of the formula (I) are allowed to act on fungi or their environment.

5. Process for regulating plant growth, characterised in that substituted triazolylmethyl-tert.-butyl-carbinols of the formula (I) or acid addition salts or metal salt complexes of substances of the formula (I) are allowed to act on the plants to be treated or their environment.

6. Use of substituted triazolylmethyl-tert.-butyl-carbinols of the formula (I) or of acid addition salts or metal salt complexes of substances of the formula (I) as plant protection agents.

7. Use of substituted triazolylmethyl-tert.-butyl-carbinols of the formula (I) or of acid addition salts or metal salt complexes of substances of the formula (I) for combating fungi.

8. Use of substituted triazolylmethyl-tert.-butyl-carbinols of the formula (I) or of acid addition salts or metal salt complexes of substances of the formula (I) for regulating plant growth.

9. Use of substituted triazolylmethyl-tert.-butyl-carbinols of the formula (I) or of acid addition salts or metal salt complexes of substances of the formula (I) as intermediate products for the preparation of plant protection agents.

10. Process for the preparation of plant protection agents, characterised in that substituted triazolylmethyl-tert.-butyl-carbinols of the formula (I) or acid addition salts or metal salt complexes of substances of the formula (I) are mixed with extenders and/or surface-active substances.

**Revendications**

1. Triazolylméthyl-tertiobutyl-carbinols substitués de formule

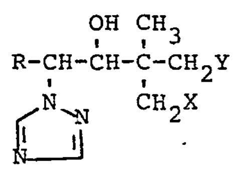

(I)

dans laquelle

R représente un groupe alkyle ayant 1 à 12 atomes de carbone, alcényle ayant 2 à 12 atomes de carbone, alcynyle ayant 2 à 12 atomes de carbone, cycloalkyle ayant 3 à 7 atomes de carbone et éventuellement substitué par un groupe alkyle ayant 1 à 4 atomes de carbone, cycloalkylalkyle comportant 3 à 7 atomes de carbone dans la partie cycloalkyle et 1 à 4 atomes de carbone dans la partie alkyle et éventuellement substitué par un groupe alkyle ayant 1 à 4 atomes de carbone, ainsi qu'un groupe aralkyle ayant 6 à 10 atomes de carbone dans la partie aryle et 1 à 4 atomes de carbone dans la partie alkyle, la partie aryle pouvant être substituée par un groupe alkyle ayant 1 à 4 atomes de carbone, alcoxy ayant 1 à 4 atomes de carbone, alkylthio ayant 1 à 4 atomes de carbone, par un atome d'halogène, un groupe halogénoalkyle ayant 1 à 4 atomes de carbone et comportant 1 à 5 atomes d'halogène identiques ou différents, halogénoalcoxy comportant 1 à 4 atomes de carbone et 1 à 5 atomes d'halogène identiques ou différents, halogénoalkylthio comportant 1 à 4 atomes de carbone et 1 à 5 atomes d'halogène identiques ou différents, nitro, cyano, alcoxycarbonyle ayant 1 à 4 atomes de carbone dans la partie alcoxy, amino, alkylamino ayant 1 à 4 atomes de carbone, dialkylamino ayant 1 à 4 atomes de carbone dans chaque partie alkyle, phénylamino, phénoxy et/ou phényle, qui peut à son tour être substituée par de l'halogène et/ou un groupe alkyle ayant 1 ou 2 atomes de carbone ;

X représente un atome d'hydrogène, et

Y représente un atome de fluor ou de chlore, ou bien

X et Y sont identiques et représentent chacun un atome de fluor ou de chlore, ainsi que leurs sels d'addition d'acides et leurs complexes avec des sels de métaux, tolérables par les plantes.

2. Procédé pour préparer des triazolylméthyl-tertiobutyl-carbinols substitués de formule

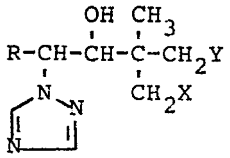

(I)

dans laquelle

R représente un groupe alkyle ayant 1 à 12 atomes de carbone, alcényle ayant 2 à 12 atomes de carbone, alcynyle ayant 2 à 12 atomes de carbone, cycloalkyle ayant 3 à 7 atomes de carbone et éventuellement substitué par un groupe alkyle ayant 1 à 4 atomes de carbone, cycloalkylalkyle comportant 3 à 7 atomes de carbone dans la partie cycloalkyle et 1 à 4 atomes de carbone dans la partie alkyle et éventuellement substitué par un groupe alkyle ayant 1 à 4 atomes de carbone, ainsi que aralkyle ayant 6 à 10 atomes de carbone dans la partie aryle et 1 à 4 atomes de carbone dans la partie alkyle, la partie aryle pouvant être substituée par un groupe alkyle ayant 1 à 4 atomes de carbone, alcoxy ayant 1 à 4 atomes de carbone, alkylthio ayant 1 à 4 atomes de carbone, par un atome d'halogène par un groupe halogénoalkyle ayant 1 à 4 atomes de carbone et 1 à 5 atomes d'halogène identiques ou différents, halogéno-alcoxy ayant 1 à 4 atomes de carbone et 1 à 5 atomes d'halogène identiques ou différents, halogénoalkylthio ayant 1 à 4 atomes de carbone et 1 à 5 atomes d'halogène identiques ou différents, nitro, cyano, alcoxycarbonyle ayant 1 à 4 atomes de carbone dans la partie alcoxy, amino, alkylamino ayant 1 à 4 atomes de carbone, dialkylamino ayant 1 à 4 atomes de carbone dans chaque partie alkyle, phénylamino, phénoxy et/ou phényle, qui peut à son tour être substituée par de l'halogène et/ou par un groupe alkyle ayant 1 ou 2 atomes de carbone,

X représente un atome d'hydrogène, et

Y représente un atome de fluor ou de chlore, ou bien

X et Y sont identiques et représentent chacun un atome de fluor ou de chlore, ainsi que leurs sels d'addition d'acides et leurs complexes avec des sels de métaux, tolérables par les plantes, procédé caractérisé en ce qu'on réduit selon des procédés connus, de façon usuelle, des triazolyl-méthyl-tertiobutyl-cétones substituées de formule

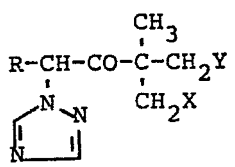

(II)

dans laquelle R, X et Y ont le sens précité, et éventuellement ensuite on fixe par addition un acide ou un sel de métal.

3. Produit phytosanitaire, caractérisé en ce qu'il contient au moins un triazolyl-méthyl-tertiobutyl-carbinol substitué de formule (I) ou un sel d'addition d'acide ou un complexe avec un sel de métal d'une substance de formule (I).

4. Procédé pour combattre des champignons, caractérisé en ce qu'on fait agir sur les champignons ou sur leur biotope ou espace vital des triazolyl-méthyl-tertiobutyl-carbinols substitués de formule (I) ou des sels d'addition d'acides ou des complexes, formés avec un sel de métal, de substances de formule (I).

5. Procédé de régulation de la croissance des plantes, caractérisé en ce qu'on fait agir sur les plantes à traiter ou sur leur biotope ou espace vital des triazolyl-méthyl-tertiobutyl-carbinols substitués de formule (I) ou des sels d'addition d'acides ou des complexes, formés avec un sel de métal, de substances de formule (I).

6. Application de triazolyl-méthyl-tertiobutyl-carbinols substitués de formule (I) ou de sels d'addition d'acides ou de complexes, formés avec un sel de métal, de substances de formule (I) comme produits phytosanitaires.

7. Application de triazolyl-méthyl-tertiobutyl-carbinols substitués de formule (I) ou de sels d'addition d'acides ou de complexes, formés avec un sel de métal, de substances de formule (I) à la lutte contre des champignons.

8. Application de triazolyl-méthyl-tertiobutyl-carbinols substitués de formule (I) ou de sels d'addition d'acides ou de complexes, formés avec un sel de métal, de substances de formule (I) à la régulation de la croissance de plantes.

9. Application de triazolyl-méthyl-tertiobutyl-carbinols substitués de formule (I) ou de sels d'addition

d'acides ou de complexes, formés avec un sel de métal, de substances de formule (I), comme produits intermédiaires pour la préparation de produits phytosanitaires.

10. Procédé de préparation de produits phytosanitaires, caractérisé en ce qu'on mélange des triazolyl-méthyl-tertiobutyl-carbinols substitués de formule (I) ou des sels d'addition d'acides ou des complexes, formés avec un sel de métal, de substances de formule (I) avec des agents d'allongement et/ou des substances tensio-actives.